(19) Europäisches Patentamt
European Patent Office
Office européen des brevets

(11) Veröffentlichungsnummer: **0 164 314**
**B1**

(12) # EUROPÄISCHE PATENTSCHRIFT

(45) Veröffentlichungstag der Patentschrift: **13.01.88**

(51) Int. Cl.⁴: **C 07 C 161/00**

(21) Anmeldenummer: **85810254.4**

(22) Anmeldetag: **03.06.85**

(54) **Sulfoxoniumsalze.**

(30) Priorität: **07.06.84 GB 8414525**

(43) Veröffentlichungstag der Anmeldung: **11.12.85 Patentblatt 85/50**

(45) Bekanntmachung des Hinweises auf die Patenterteilung: **13.01.88 Patentblatt 88/2**

(84) Benannte Vertragsstaaten: **BE CH DE FR GB IT LI NL SE**

(56) Entgegenhaltungen: **US-A-4 398 014**

(73) Patentinhaber: **CIBA- GEIGY AG, Klybeckstrasse 141, CH- 4002 Basel (CH)**

(72) Erfinder: **Banks, Christopher Paul, 105 Ross Close Saffron Walden, Essex CB11 4DU (GB)**
Erfinder: **Irving, Edward, Dr., 41, Swaffham Road Burwell, Cambridge CB5 0AN (GB)**

**EP 0 164 314 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft neue Sulfoxoniumsalze und deren Verwendung als thermische oder photochemische Polymerisationskatalysatoren.

Unterschiedlichste Sulfoxoniumsalze sind als Katalysatoren für die thermische und/oder photochemische Polymerisation kationisch polymerisierbarer Materialien vorgeschlagen worden. Diese vorbekannten Sulfoxoniumsalze besitzen wenigstens eine aromatische Gruppe im Kation. Solche Verbindungen sind beispielsweise in den US-PSS 4 299 938, 4 339 567, 4 383 025 oder 4 398 014 beschrieben worden.

Obgleich Sulfoxoniumsalze mit mindestens einer aromatischen Gruppe im Molekül als thermische und/oder photochemische Polymerisationskatalysatoren wirken, ist ihre Anwendbarkeit in einigen Fällen begrenzt.

Setzt man beispielsweise ein solches aromatisches Sulfoxoniumsalz bei der Photopolymerisation eines aromatischen Harzes ein, beispielsweise eines aromatischen Epoxidharzes, so absorbieren Harz und Photokatalysator gegebenenfalls bei derselben Wellenlänge; Licht dieser Wellenlänge wird aber zur Aktivierung des Katalysators benötigt.

Da der Katalysator jedoch in der Regel nur in kleiner Menge, beispielsweise etwa 3 Gew.-% vorliegt, wird das Harz den grössten Anteil des Liches absorbieren und die Menge an Lichtquanten, die zur Aktivierung des Katalysators zur Verfügung steht, wird beträchtlich reduziert.

Unter diesen Umständen kann es vorkommen, dass nur eine sehr dünne Harzschicht gehärtet werden kann, oder dass überhaupt keine Härtung eintritt.

Es besteht also ein Bedarf nach einem Katalysator, dessen Absorptionsspektrum sich von dem des Harzes unterscheidet und der es gestattet, dickere Filme auszuhärten.

In diesem Zusammenhang ist weiterhin wichtig, dass eine Tendenz zur Verwendung von immer kurzwelligerem Ultraviolettlicht beim Belichten von Photolacken besteht. Der Einsatz des kurzwelligeren Lichtes gestattet die Abbildung höher aufgelöster Muster, und daher können elektrische Schaltkreise mit einer grösseren Flächendichte an Schaltelementen hergestellt werden. Es ist also auch unter diesem Aspekt wünschenswert, einen Katalysator zur Verfügung zu haben, der bei kurzen Wellenlängen aktivierbar ist.

Bei vielen Anwendungen werden Harzgemische eingesetzt, um dem Produkt ein Optimum an Eigenschaften zu verleihen, beispielsweise bei Metallbeschichtungsverfahren, wie sie bei der Dosenherstellung üblich sind. In der Regel wird für jedes der Harze ein separater Katalysator benötigt. Der Herstellungsprozess würde vereinfacht werden, wenn man einen Katalysator zur Verfügung hätte, der für jedes der eingesetzten Harze verwendbar wäre.

Es wurde nun gefunden, dass gewisse aliphatische Sulfoxoniumsalze sich als thermische und photochemische Katalysatoren der Polymerisation von Epoxidharzen, bevorzugt aromatischen Epoxidharzen, von Vinylharzen oder anderen Harzen eignen.

Die vorliegende Erfindung betrifft aliphatische Sulfoxoniumsalze der Formel I

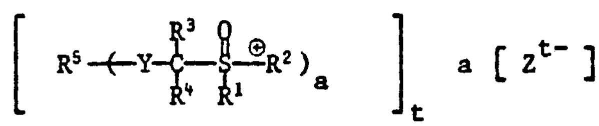 (I),

worin $R^1$ $C_1$-$C_{18}$ Alkyl, $C_2$-$C_6$ Alkenyl, $C_3$-$C_8$ Cycloalkyl oder $C_4$-$C_{10}$ Cycloalkylalkyl bedeutet,

$R^2$ eine der Bedeutungen von $R^1$ besitzt oder zusätzlich $C_2$-$C_6$ Dialkylamino ist, worin $R^3$ und $R^4$ unabhängig voneinander Wasserstoff, $C_1$-$C_{25}$ Alkyl oder eine der Gruppen $-COR^6$, $-CO-NH-(CO)_b-R^6$ oder $-SO_2-R^6$ darstellen, worin

$R^6$ gesättigtes oder ethylenisch ungesättigtes $C_1$-$C_{25}$ Alkyl bedeutet, worin

$R^5$ ein mono- bis tetravalenter gesättigter oder ungesättigter aliphatischer $C_1$-$C_{25}$ Rest ist, ein cycloaliphatischer Rest mit bis zu 25 Kohlenstoffatomen ist oder einen $C_1$-$C_{25}$ Alkoxyrest darstellt, worin

Y eine Carbonylgruppe, eine Sulfonylgruppe oder einen Rest der Formel $-(CO)_b-NH-CO-$ darstellt,

a eine ganze Zahl von 1 bis 4 ist,

b 0 oder 1 bedeutet,

t 1, 2 oder 3 ist,

$Z^{t-}$ ein t-wertiges Anion ist, und ein Hydroxypentafluorantimonatanion ist, oder ein Anion der Formel $R^7-(SO_3^-)_t$ oder $MX_c^-$,

$R^7$ Wasserstoff oder ein ein-, zwei- oder dreiwertiger aliphatischer, aromatischer oder aliphatisch-substituierter aromatischer $C_1$-$C_{25}$ Rest ist,

M ein Metall oder Metalloidatom bedeutet,

X ein Halogenatom ist, und worin schliesslich

c 4, 5 oder 6 ist, und um eins grösser ist, als die Valenz von M.

$R^1$ und $R^2$ bedeuten vorzugsweise gleichzeitig $C_1$-$C_4$ Alkyl, und ganz besonders bevorzugt Methyl.

$R^3$ und $R^4$ sind vorzugsweise Wasserstoff oder $C_1$-$C_4$ Alkyl, ganz besonders bevorzugt jedoch Wasserstoff.

$R^5$ ist vorzugsweise $C_1$-$C_4$ Alkyl, wie beispielsweise Methyl, Ethyl oder n-Butyl; oder $R^5$ ist bevorzugt ein cycloaliphatischer Rest mit bis zu 12 C-Atomen, wie beispielsweise Cyclohexyl oder Tricyclo-[3.3.1.1,$^{3,7}$] decan; oder $R^5$ ist vorzugsweise $C_1$-$C_4$ Alkoxy, wie beispielsweise Methoxy, Ethoxy, Propoxy oder Butoxy.

$R^7$ ist vorzugsweise Wasserstoff, Methyl, Trifluormethyl, Phenyl, Tolyl, 1,4-Phenylen oder der Rest eines

sulfonierten Phenol-Formaldehyd Novolaks nach dem Entfernen von 1, 2 oder 3 Sulfonsäuregruppen.

Spezifische Besispiele von Anionen $Z^{t-}$ sind
$HSO^-_3$, $CF_3SO^-_3$, $C_6H_5SO^-_3$, $CH_3C_6H_4SO^-_3$, $^-O_3SC_6H_4SO^-_3$ und
$^-O_3S\text{-}C_6H_3(OH)\text{-}CH_2\text{-}C_6H_2(OH)(SO^-_3)\text{-}CH_2\text{-}(OH)C_6H_3\text{-}SO^-_3$.

M ist vorzugaweise ein Atom ausgewählt aus der Gruppe der Elemente Bor, Wismut, Antimon, Arsen oder Phosphor. Weitere spezifische Beispiele von Anionen $Z^{t-}$ sind $BiCl^-_6$, $BF^-_4$, $PF^-_6$, $SbF^-_6$ oder $AsF^-_6$.

Die aliphatischen Sulfoxoniumsalze der vorliegenden Erfindung lassen sich beispielsweise erhalten, indem nan ein Oxosulfonium-Ylid der Formel II

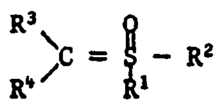

(II)

mit

a) einem Säurehalogenid der Formel III

$$R^5 - (- CO\text{-}Hal)_a \qquad (III)$$

oder mit einem Anhydrid einer Säure der Formel IV umsetzt

$$R^5 - (- COOH)_a \qquad (IV);$$

oder man setzt das Ylid der Formel II mit

b) einem Sulfonylhalogenid der Formel V, vorzugsweise einem Fluorid oder Chlorid, um

$$R^5 - (- SO_2\text{-}Hal)_a \qquad (V);$$

oder man setz das Ylid der Formel II mit

c) einem Isocyanat der Formel VI um

$$R^5 - [- (CO)_b\text{-}NCO]_a \qquad (VI),$$

und neutralisiert anrantimonate, auch durch doppelte Umsetzung der entsprechenden Chloride oder anderer geeigneter Salze herstellen.

In einem solchen Fall kann man also das Reaktionsprodukt der Umsetzung von a), b) oder c) mit irgend einer anderen Säure neutralisieren, beispielsweise mit Salzsäure und anschliessend das resultierende Salz mit einem Metall- oder Ammoniumsalz des Anions $Z^{t-}$ umsetzen.

Stellt man ein Sulfoxoniumsalz I durch Umsetzung nach Reaktion a) her, so verwendet man vorzugsweise ein Anhydrid der Säure der Formel IV.

Jede der oben beschriebenen Reaktionen wird vorzugsweise so ausgeführt, indem man den betreffenden Reaktanden langsam dem Ylid zugibt.

Die Ylide der Formel II lassen sich beispielsweise nach der Methode von Corey und Chaykovsky (J. Am. Chem. Soc., 84, 867 (1962)) herstellen. Bei diesem Verfahren wird ein Sulfoxoniumhalogenid, beispielsweise ein Chlorid oder ein lodid der Formel VII

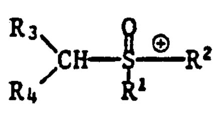

$Hal^{\ominus}$ (VII)

mit einer starken Base, wie beispielsweise mit Natriumhydrid, umgesetzt.

Die erfindungsmässen Verbindungen sind nützliche Polymerisationskatalysatoren für Verbindungen, die sich unter dem Einfluss eines Katalysators des kationischen oder der radikalischen Polymerisation umsetzen.

Die Erfindung betrifft daher auch eine Zusammensetzung enthaltend

a) eine Verbindung, die sich unter dem Einfluss des Salzes der Formel I polymerisieren lässt und

b) eine wirksame Menge eines aliphatischen Sulfoxoniumsalzes der Formel I wie oben definiert.

Die Menge der Komponente b) in den erfindungsgemässen Zusammensetzungen muss ausreichen, um die Polymerisation von a) unter der Einwirkung von Hitze oder aktinischer Strahlung auszulösen. In der Regel verwendet man zwischen 0,1 und 7,5, vorzugsweise von 0,5 bis 6, Gewichtsteile von b) auf 100 Gewichtsteile a).

Bei der Komponente a) handelt es sich vorzugsweise um ein 1,2-Epoxid, ein Vinylmonmeres oder dessen

Präpolymeres, einen Aminoplast oder einen Phenoplast.

Dabei handelt es sich beispielsweise um ein Mono-1,2-epoxid, wie Epichlorhydrin, Propylenoxid, den Glycidylether eines einwertigen Alkohols oder Phenols, wie beispilsweise den n-Butylglycidylether oder den Phenylglycidylether; es kann sich aber auch um einen Glycidylester, wie beispielsweise das Glycidylacrylat oder das Glycidylmethacrylat handeln. Andere geeignete Komponenten sind epoxidfreie Ester der Acrylsäure oder der Methacrylsäure.

Vorzugsweise handelt es sich bei der Komponente a) um ein Epoxidharz, insbesondere um solche Harze enthaltend wenigstens eine Gruppe der Formel VIII

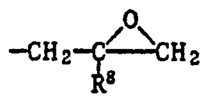

(VIII),

wobei diese Gruppe direkt an ein Sauerstoffatom gebunden ist, und worin $R^8$ Wasserstoff oder Methyl bedeutet.

Beispiele für solche Harze sind Polyglycidyl- und Poly-(β-methylglycidyl)-ester erhältlich durch Umsetzung einer Verbindung mit zwei oder mehreren Carboxylgruppen im Molekül und Epichlorhydrin, Glycerindichlorhydrin oder β-Methylepichlorhydrin in Gegenwart von Basen.

Solche Polyglycidylester können sich von aliphatischen Polycarbonsäuren ableiten, beispielsweise von Bernsteinsäure, Glutarsäure, Adipinsäure, Pimelinsäure, Korksäure, Azelainsäure, Sebazinsäure oder dimerisierter bzw. trimerisierter Linolsäure; sie können sich aber auch von cycloaliphatischen Polycarbonsäuren ableiten, wie beispielsweise von Tetrahydrophthalsäure, 4-Methyltetrahydrophthalsäure, Hexahydrophthalsäure oder 4-Methylhexahydrophthalsäure; sie können sich weiterhin von aromatischen Polycarbonsäuren ableiten, wie beispielsweise von Phthalsäure, Isophthalsäure oder Terephthalsäure.

Weitere geeignete Polyglycidylester sind durch Vinylpolymerisation von Glycidylestern der Vinylsäuren erhältlich, insbesondere durch Polymerisation von Glycidylacrylat oder Glycidylmethacrylat.

Weitere Beispiele für Komponenten a) sind Polyglycidyl- und Poly(β-methylglycidyl)-ether erhältlich durch Reaktion einer Verbindung enthaltend mindestens zwei freie alkoholische Hydroxygruppen und/oder phenolische Hydroxylgruppen pro Molekül mit einem geeigneten Epichlorhydrin unter alkalischen Bedingungen, oder in Gegenwart eines sauren Katalysators und nachfolgender Behandlung mit Basen.

Diese Ether leiten sich beispielsweise ab von acyclischen Alkoholen wie Ethylenglykol, Diethylenglykol, oder höheren Poly-(oxyethylen)-glykolen, Propan-1,2-diol oder Poly-(oxypropylen)-glykolen, Propan-1,3-diol, Poly-(oxytetramethylen)-glykolen, Pentan-1,5-diol, Hexan-2,4,6-triol, Glycerin, 1,1,1-Trimethylolpropan, Pentaerythrit, Sorbit oder Poly-(epichlorhydrin); von cycloaliphatischen Alkoholen wie z. B. 1,3- oder 1,4-Dihydroxycyclohexan, Bis-(4-hydroxycyclohexyl)-methan, 2,2-Bis-(hydroxycyclohexyl)-propan und 1,1-Bis-(hydroxymethyl)-cyclohex-3-en; sowie von Alkoholen mit aromatischen Kernen, wie z. B. N,N-Bis-(2-hydroxyethyl)-anilin und p,p'-Bis-(2-hydroxyethylamino)-diphenylmethan. Oder sie leiten sich von einkernigen Phenolen ab, wie z. B. Resorcin oder Hydrochinon oder von mehrkernigen Phenolen, wie z. B. Bis-(4-hydroxyphenyl)-methan, 4,4'-Dihydroxydiphenyl, Bis-(4-hydroxyphenyl)-sulfon, 1,1,2,2-Tetra-kis-(4-hydroxyphenyl)-ethan, 2,2-Bis-(4-hydroxyphenyl)-propan (Bisphenol A), 2,2-Bis-(3,5-dibrom-4-hydroxyphenyl)-propan und Novolaken erhältlich aus Aldehyden wie z. B. Formaldehyd, Acetaldehyd, Chloral und Furfuraldehyd mit Phenol bzw. mit Phenolen, die mit Ring durch Chloratome oder Alkylgruppen substituiert sind, wobei die Alkylgruppen bis zu neun Kohlenstoffatome tragen können. Bei diesen Phenolen handelt es sich beispielsweise um 4-Chlorphenol, 2-Methylphenol oder 4-tert.Butylphenol.

Man kann als Komponente a) auch Poly-(N-glycidyl)-verbindungen einsetzen; dazu zählen beispielsweise N-Glycidylderivate von Aminen, wie z. B. von Anilin, n-Butylamin, Bis-(4-aminophenyl)-methan und Bis-(4-methylaminophenyl)-methan; ferner zählen dazu Triglycidylisocyanurate, oder auch N,N'-Diglycidylderivate von cyclischen Alkylenharnstoffen, wie z. B. von Ethylenharnstoff oder 1,3-Propylenharnstoff, oder auch von Hydantoinen, wie z. B. von 5,5-Dimethylhydantoin. In der Regel sind diese Verbindungen jedoch nicht bevorzugt.

Weitere Verbindungen, die als Komponente a) in den erfindungsgemässen Zusammensetzungen eingesetzt werden können, sind Poly(S-glycidyl)-derivate. Dabei handelt es sich beispielsweise um Di-(S-glycidyl)-derivate von Dithiolen, wie beispielsweise von Ethan-1,2-dithiol und Bis-(4-merkaptomethylphenyl)-ether. Aber auch diese Verbindungen sind in der Regel nicht bevorzugt.

Epoxidharze mit 1,2-Epoxidgruppen, die an unterschiedliche Heteratome gebunden sind, können ebenfalls eingesetzt werden. Dazu zählen beispielsweise der Glycidylether-ester von p-Hydroxybenzoesäure.

Auch können Epoxidharze verwendet werden, bei denen einige oder alle Epoxidgruppen nicht endständig sind. Dazu zählen beispielsweise Vinylcyclohexendioxid, Limonendioxid, Dicyclopentadiendioxid,

4-Oxatetracyclo-[6.2.1.0$^{2,7}$.0$^{3,5}$]-undec-9-ylglycidylether,

1,2-Bis-(4-oxatetracyclo[6.2.1.0$^{2,7}$.0$^{3,5}$]-undec-9-yloxyl)-ethan,

3,4-Epoxycyclohexylmethyl-3',4'-epoxycyclohexancarboxylat und sein 6,6'-Dimethylderivat, Ethylenglykol-bis-(3,4-epoxycyclohexan-carboxylat), 3-(3,4-Epoxycyclohexyl)-8,9-epoxy-2,4-dioxaspiro-[5,5]-undecan, und epoxidierte Polybutadiene oder Copolymere von Butadien mit ethylenisch ungesättigten Verbindungen wie Styrol und Vinylacetat.

Falls gewünscht, können auch Mischungen von Epoxidharzen eingesetzt werden. Besonders bevorzugte Epoxidharze im Rahmen dieser Erfindung sind Epoxidverbindungen, enthaltend wenigstens einen aromatischen Ring, wie beispielsweise Diglycidylether, die gegebenenfalls vorverlängert sind, von zweiwertigen Phenolen, wie beispielsweise von 2,2-Bis-(4-hydroxyphenyl)-propan oder von Bis-(4-hydroxyphenyl)-methan.

Falls gewünscht, kann das Epoxidharz zusammen mit einem polyvalenten Alkohol gehärtet werden. Zu diesen Alkoholen zählen beispielsweise Verbindungen mit wenigstens zwei alkoholischen Hydroxylgruppen, vorzugsweise primären Hydroxylgruppen, im Molekül.

Vorzugsweise ist der polyvalente Alkohol in Mengen anwesend, die genügen, um 0,5 bis 1,5, vorzugsweise 0,75 bis 1,25, 1,2-Epoxidgruppen des Epoxidharzes abzusättigen.

Vorzugsweise enthält der polyvalente Alkohol zusätzlich zu den alkoholischen Hydroxygruppen nur die Elemente Kohlenstoff und Wasserstoff, sowie gegebenenfalls Sauerstoff, der dann als Ethersauerstoff vorliegt, sowie Acetalgruppen, Carbonyloxygruppen oder Halogenatome.

Weiterhin wird bevorzugt, dass der polyvalente Alkohol ein Molekulargewicht von wenigstens 100 und insbesondere von mehr als 1,000 besitzt.

Beispiele für besonders geeignete Alkohole dieses Typs sind Poly(oxyethylen)-glykole, Poly-(oxypropylen)-glykole, Poly-(oxytetramethylen)-glykole, Polyepichlorhydrine, Poly-(oxyethylen)-, Poly(oxypropylen)-, oder Poly-(oxytetramethylen)-triole, erhältlich durch Polymerisation von Ethylenoxid, Propylenoxid oder Tetrahydrofuran in Gegenwart von Glycerin oder 1,1,1-Trimethylolpropan, sowie hydroxyl-terminierte Polycaprolactone, Copolymere von Styrol mit Allylalkohol, Polyvinylalkohole, Hydroxypropylcellulose, hydroxyhaltige Polyvinylacetale und Partialester von Cellulose, z. B. das Celluloseacetatbutyrat.

Vinylmonomere und Präpolymere, die polymerisiert werden können, umfassen beispielsweise Styrol, α-Methylstyrol, Allylbenzol, Divinylbenzol, Vinylcyclohexan, 4-Vinylcyclohex-1-en, N-Vinyl-pyrrolidin-2-on, N-Vinylcarbazol, Acrolein, Isopren, Butadien, Piperylene, Vinylacetat und Vinylether wie z. B. Isobutylvinylether, Methylvinylether, Trimethylolpropantrivinylether, Glycerintrivinylether, Vinylether von Ethylenglykol und Poly-(oxyethylenglykolen), sowie cyclische Vinylether mit mindestens zwei cyclischen Vinylethergruppen, die, jede für sich, den Teil eines 3,4-Dihydro-2H-pyranringes bilden, wie beispielsweise 3,4-Dihydro-2H-pyran-2-ylmethyl-3,4-dihydro-2H-pyran-2-carboxylat und dessen Präpolymere.

Bevorzugt unter diesen Vinylverbindungen werden Vinylether von aliphatischen monovalenten Alkoholen sowie 3,4-Dihydro-2H-pyran-2-ylmethyl-3,4-dihydro-2H-pyran-2-carboxylat und dessen Präpolymere.

Weitere geeignete Vinylmonomere und deren Präpolymere stellen Ester der Acrylsäure bzw. der Methacrylsäure dar, vorzugsweise solche mit zwei oder mehr Acrylat- bzw. Methacrylatresten im Molekül.

Vorzugsweise handelt es sich bei diesen Estern um Verbindungen, die sich von Glykolen ableiten, beispielsweise von Ethylenglykol, Triethylenglykol oder Tetraethylenglykol; oder es handelt sich um Ester, die durch Umsetzung von Acrylsäure oder Methacrylsäure mit Mono- oder Polyglycidylethern von mono- oder polyvalenten Alkoholen oder von Phenol oder mit einem N-Glycidylhydantoin entstehen.

Weitere geeignete Ester bilden sich bei der Umsetzung eines Adduktes eines Hydroxyalkylacrylates oder -methacrylates an ein Anhydrid, vorzugsweise an Phthalsäure-, Bernsteinsäure- oder Maleinsäureanhydrid mit einem Epoxidharz.

Zu den typischen Estern dieser Art zählen beispielsweise 1,4-Bis-(2-hydroxy-3-acryloyloxypropoxy)-butan, der Poly-(2-hydroxy-3-acryloyloxypropyl)-ether eines Phenolformaldehydnovolaks, 2,2-Bis-(4-(2-hydroxy-3-acryloyloxypropoxy)-phenyl)-propan, 2,2-Bis-(4-(2-hydroxy-3-(2-acryloyloxyethoxy)-succinyloxypropxy)-phenyl)-propan, 1-(2-Hydroxy-3-acryloyloxy-propoxy)-butan, -octan und -decan, Bis-(2-hydroxy-3-acryloyloxy-propyl)-adipat, 2-Hydroxy-3-acryloyloxypropylpropionat und 3-Phenoxy-2-hydroxy-propylacrylat, sowie die entsprechenden Methacrylate.

Zu den Aminoplasten, die als Komponente a) in den erfindungsgemässen Zusammensetzungen verwendet werden können, zählen vorzugsweise Verbindungen, die pro Molekül wenigstens zwei Gruppen der Formel $-CH_2-OR^{11}$ enthalten, wobei diese Gruppen direkt an ein Amid- oder Thioamidstickstoffatom gebunden sind, worin $R^{11}$ Wasserstoff, $C_1$-$C_4$ Alkyl oder Acetyl bedeutet.

Beispiele für Verbindungen dieses Typs sind die N-Hydroxymethyl-, N-Methoxymethyl-, N-Butoxymethyl- und N-Acetoxymethylderivate der folgenden Amide oder amidähnlichen Verbindungen.

I. Harnstoff, Thioharnstoff und die cyclischen Derivate der Formel IX

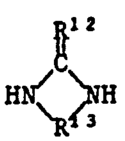

(IX),

worin $R^{12}$ Sauerstoff oder Schwefel ist, worin $R^{13}$ entweder eine Gruppe der Formel X ist

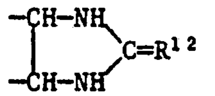 (X),

oder eine divalente Gruppe darstellt, die durch Methyl, Methoxy oder Hydroxy substituiert sein kann, und die durch -CO-, -O- oder $-N(R^{14})-$ unterbrochen sein kann, worin $R^{14}$ Alkyl oder Hydroxyalkyl mit bis zu 4 Kohlenstoffatomen bedeutet und worin besagte divalente Gruppe zwei bis vier Kohlenstoffatome enthält, abgesehen von Kohlenstoffatomen in allfälligen Methyl oder Methoxysubstituenten oder in -CO- oder $-N(R^{14})-$ Resten.

Beispiele für die obengenannten cyclischen Harnstoffe sind Ethylenharnstoff (Imidazolidin-2-on), Dihydroxyethylenharnstoff (4,5-Dihydroxyimidazolidin-2-on), Hydantoin, Uron (Tetrahydrooxadiazin-4-on), 1,2-Propylenharnstoff (4-Methylimidazolidin-2-on), 1,3-Propylenharnstoff (Hexahydro-2H-pyrimid-2-on), Hydroxypropylenharnstoff (5-Hydroxyhexahydro-2H-pyrimid-2-on), Dimethylpropylenharnstoff (5,5-Dimethylhexahydro-2H-pyrimid-2-on), Dimethylhydroxypropylenharnstoff und Dimethylmethoxypropylenharnstoff (4-Hydroxy- und 4-Methoxy-5,5-dimethylhexahydro-2H-pyrimid-2-on), 5-Ethyltriazin-2-on und 5-(2-Hydroxyethyl)-triazin-2-on.

II. Carbamate und Dicarbamate von aliphatischen ein- und zweiwertigen Alkohlen enthaltend bis zu vier Kohlenstoffatome. Dazu zählen beispielsweise Methyl-, Ethyl-, Isopropyl-, 2-Hydroxyethyl-, 2-Methoxyethyl-, 2-Hydroxy-n-propyl- und 3-Hydroxy-n-propyl-carbamate, sowie Ethylen- oder 1,4-Butylendicarbamate.

III. Melamin und andere Polyamino-1,3-triazine, wie beispielsweise Acetoguanamin, Benzoguanamin oder Adipoguanamin.

Falls gewünscht, können Aminoplaste enthaltend sowohl N-Hydroxymethylgruppen als auch N-Alkoxymethylgruppen oder enthaltend sowohl N-Hydroxymethylgruppen als auch N-Acetoxymethylgruppen eingesetzt werden; dabei handelt es sich beispielsweise um ein Hexamethylolmelamin, in dem ein bis drei der Hydroxylgruppen mit Methylresten verethert sind. Bevorzugte Aminoplaste sind Kondensationsprodukte von Harnstoff, Uron, Hydantoin oder Melamin mit Formaldehyd, und die teilweise oder vollständig veretherten Produkte dieser Kondensationsprodukte, mit einem aliphatischen einwertigen Alkohol enthaltend ein bis vier Kohlenstoffatome.

Zu den bevorzugten Phenoplasten im Rahmen dieser Erfindung zählen Resole, die sich von einem Phenol und einem Aldehyd ableiten. Geeignete Phenole umfassen Phenol, Resorcin, 2,2-Bis-(p-hydroxyphenyl)-propan, p-Chlorphenol, Phenole, die durch eine oder zwei Alkylgruppen mit einem Gehalt von ein bis neun Kohlenstoffatomen substituiert sind, so wie beispielsweise o-, m- oder p-Kresol, die Xylenole, p-tert.Butylphenol, p-Nonylphenol, sowie phenylsubstituierte Phenole, wie beispielsweise p-Phenylphenol.

Der Aldehyd, der mit dem Phenol kondensiert wird, ist vorzugsweise Formaldehyd. Es lassen sich aber auch andere Aldehyde, wie beispielsweise Acetaldehyd oder Furfuraldehyd, verwenden. Falls gewünscht, kann auch eine Mischung solcher härtbarer Phenol-Aldehydharze eingesetzt werden.

Ganz besonders bevorzugte Resole sind Kondensationsprodukte von Phenol, p-Chlorphenol oder o-, m- oder p-Kresol mit Formaldehyd.

Die Photopolymerisation der erfindungsgemässen Zusammensetzungen erfolgt zweckmässigerweise mit aktinischer Strahlung einer Wellenlänge von 180 bis 600 nm.

Geeignete Strahlungsquellen sind beispielsweise Kohlebogenlampen, Quecksilberdampflampen, Leuchtstoffröhren mit UV-Licht emittierenden Leuchtstoffen, Argon- oder Xenon-Glühentladungslampen, Wolframlampen, sowie Lampen für photographisches Flutlicht. Von diesen Lichtquellen sind insbesondere Quecksilberdampflampen, vor allem solche, die das Sonnenspektrum emittieren, bevorzugt; weiterhin bevorzugt sind fluoreszierende Sonnenlichtlampen oder Metallhalogenglühlampen.

Die Belichtungsdauer hängt im Einzelfall von einer Vielzahl von Faktoren ab; dazu zählen beispielsweise die einzelnen zu polymerisierenden Verbindungen, die Art der Strahlungsquelle sowie deren Abstand vom zu belichtenden Substrat.

Geeignete Belichtungszeiten lassen sich vom Fachmann anhand von Routinetests leicht ermitteln.

Die Photopolymerisation kann durch Erwärmen der Probe während der Bestrahlungsdauer unterstützt werden.

Das photopolymerisierbare Material besitzt vorzugsweise eine aromatische Gruppe im Molekül und/oder ein aromatischer Sensibilisator wird verwendet und/oder harte UV-Strahlung wird verwendet, insbesondere Strahlung mit einer Wellenlänge zwischen 180 und 260 nm. Zu den aromatischen Sensibilisatoren zählen vorzugsweise aromatische Verbindungen mit Triplett-Energien von über 75 kcal/Mol. Dazu zählen beispielsweise Phenole, wie beispielsweise Phenol, Resorcin, 2,6-Di-tert-butylphenol, m-Kresol, p-Kresol oder Phenol-Formaldehyd Novolake, oder Phenolether, wie beispielsweise Anisol, oder halogenierte aromatiache Verbindungen, wie beispielsweise 1,4-Dibrombenzol.

Sollen die erfindungsgemässen Zusammensetzungen hauptsächlich mittels Wärmezufuhr polymerisiert werden, so erhitzt man sie vorzugsweise auf Temperaturen von 100 bis 175°C, bis die Polymerisation erfolgt ist. Im Falle der thermischen Polymerisation ist es von Vorteil, wenn dem Sulfoxoniumsalz ein Aktivator beigefügt wird.

Geeignete Aktivatoren sind beispielsweise Salze oder Komplexe von Übergangsmetallen, Zinnsalze,

organische Peroxide oder aktivierte α-Hydroxyverbindungen.

Zu den organischen Peroxiden, die sich als Aktivator in den erfindungsgemässen härtbaren Zusammensetzungen verwenden lassen, zählen beispielsweise Dicumylperoxid, tert.-Butylperbenzoat, tert.Butylperoxid und insbesondere Benzoylperoxid. Als Zinnsalz verwendet man vorzugsweise Zinnchlorid.

Bei dem Aktivator handelt es sich vorzugsweise um ein Salz oder einen Komplex eines Übergangsmetalls oder um eine aktivierte α-Hydroxyverbindung.

Bei den Übergangsmetallen handelt es sich um Elemente der ersten Übergangsreihe von Scandium bis Zink und um solche der zweiten Übergangsreihe von Yttrium bis Cadmium. Zu den bevorzugten Übergangsmetallen dieser Gruppe, deren Salze oder Komplexe im Rahmen dieser Erfindung Verwendung finden, zählen Zink, Kobalt, Chrom, Eisen und, ganz besonders bevorzugt, Kupfer.

Bei den Salzen dieser Elemente handelt es sich beispielsweise um Salze von organischen oder anorganischen Säuren, wie beispielsweise um die Chloride, Acetate, Trichloracetate, Naphthenate, Octanoate oder Oxalate.

Bei den Komplexen dieser Elemente handelt es sich beispielsweise um π-Orbitalkomplexe, die mit Liganden gebildet werden, welche innere Komplexe bilden können; dazu zählen beispielsweise Aldehyde, Ketone, Carboxamide, sowie aliphatische Amino-Monocarbonsäuren oder Aminopolycarbonsäuren.

Besonders bevorzugte Komplexe sind diejenigen, die mit 1,3-Diketonen, wie beispielsweise Acetylaceton, gebildet werden, oder mit dessen Homologen, beispielsweise mit Benzoylaceton, oder mit anderen Derivaten, beispielsweise mit Ethylacetoacetat.

Bei den aktivierten α-Hydroxyverbindungen handelt es sich um Substanzen, die eine Hydroxygruppe an einem Kohlenstoffatom aufweisen, welches seinerseits in α-Position zu einer aktivierenden Gruppe steht. Solche aktivierenden Gruppen sind beispielaweise Carbonylgruppen oder Kohlenstoffatome mit einer Hydroxylgruppe. Verbindungen dieses Typs bilden beim Erwärmen freie Radikale.

Zu geeigneten aktivierten α-Hydroxyverbindungen zählen beispielsweise Ascorbinsäure, oder Ketone, wie Acyloine oder Benzoine, oder aktivierte Diole, wie Pinacol und dessen Homologe, insbesondere Benzpinacol.

Falls gewünscht, können mehrere Aktivatoren gleichzeitig eingesetzt werden. So lassen sich beispielsweise ein Übergangsmetallsalz oder ein Übergangsmetallkomplex, wie Kupferacetylacetonat oder Kupferbenzoat, zusammen mit Ascorbinsäure oder mit Benzpinacol einsetzen.

Die einzusetzende Menge des Aktivators ist nicht kritisch. Sie beläuft sich in der Regel auf 10 bis 150 Gew.-%, bezogen auf die Menge an eingesetztem Sulfoxoniumsalz.

Die erfindungsgemässen Zusammensetzungen lassen sich beispielsweise als Oberflächenbeschichtungen einsetzen. Sie können auf Substraten, wie beispielsweise Stahl, Aluminium, Kupfer, Cadmium, Zink, Papier oder Holz aufgebracht werden, insbesondere als flüssige Formulierungen, die anschliessend erhitzt und/oder bestrahlt werden.

Es lassen sich auch nur Teile der Oberflächenbeschichtung polymerisieren, indem man beispielsweise die Bestrahlung durch eine Photomaske durchführt. Die unbestrahlten Teile können anschliessend mit einem geeigneten Lösungsmittel ausgewaschen werden. Dabei werden nicht polymerisierte Anteile des Beschichtungsmaterials entfernt, während die photopolymerisierten unlöslichen Teile zurückbleiben. Die erfindungsgemässen Zusammensetzungen lassen sich daher auch bei der Herstellung von Druckplatten und von gedruckten Schaltungen verwenden. Verfahren zur Herstellung von Druckplatten und gedruckten Schaltungen unter Zuhilfenahme von photopolymerisierbaren Materialien sind eine wohlbekannte Technik.

Enthalten die erfindungsgemässen Zusammensetzungen ein Material, das unter dem Einfluss eines Salzes der Formel I härtbar ist, beispielsweise ein Epoxidharz oder einen Phenoplast, oder ein anderes wärmehärtbares Material, so können diese Zusammensetzungen durch den Einfluss von aktinischer Stahlung vorgehärtet werden und anschliessend durch Erhitzen endgültig ausgehärtet werden.

Soll ein solcher Zweistufenprozess durchgeführt werden, so enthält die Zusammensetzung vorzugsweise einen Aktivator, wie oben beschrieben, zur Verbesserung der thermischen Härtung durch das Sulfoxoniumsalz, oder die Zusammensetzung enthält einen zusätzlichen wärmeaktivierbaren Härter für das härtbare Material.

Solche wärmeaktivierbaren Härter sind wohlbekannt, und der Fachmann auf dem Gebiet der wärmehärtbaren Harze kann solche Härter für ein besonderes Harz mittels Routinetests auswählen.

Falls gewünscht wird, dass ein teilweise ausgehärtetes Material für eine nachfolgende Verfahrensstufe wärmehärtbar bleiben soll, ist es natürlich wichtig, den ersten Schritt der Strahlungspolymerisation bei einer so tiefen Temperatur auszuführen, dass eine wesentliche Aktivierung des wärmeaktivierbaren Härters unterbleibt.

Die Zusammensetzungen dieser Erfindung enthaltend Epoxidharze oder Phenoplaste lassen sich also erforderlichenfalls in zwei Stufen aushärten.

Handelt es sich bei dem Phenoplast um einen Novolak, der unter dem Einfluss eines Sulfoxoniumsalzes der Formel I nicht polymerisiert, so wird dieser zusammen mit einem Material eingesetzt, das unter dem Einfluss besagten Sulfoxoniumsalzes polymerisiert; bei diesem Material handelt es sich beispielsweise um ein Epoxidharz, ein Acrylat oder ein Methacrylat. Die erfindungsgemässe Zusammensetzung wird vorzugsweise zuerst durch teilweises Härten in eine B-Stufe überführt; dazu wird die Zusammensetzung aktinischer Strahlung ausgesetzt, wobei ein latentes wärmeaktivierbares Vernetzungsmittel für das Epoxidharz oder den Phenoplasten (fakultativ, falls der Phenoplast ein Resol ist) anwesend ist. Anschliessend wird die teilweise ausgehärtete Zusammensetzung in einer zweiten Stufe erhitzt, so dass die Härtung vervollständigt wird.

Auf diese Weise ist es möglich, eine flüssige oder halbflüssige Zusammensetzung herzustellen, die verformt

werden kann oder zum Imprägnieren eines Substrates verwendet werden kann, und die sich unter dem Einfluss von Strahlung verfestigen lässt und anschliessend, falls gewünscht, erhitzt werden kann, um die Härtung des Harzes zu vervollständigen.

Zu den geeigneten wärmeaktivierbaren Vernetzungsmitteln für Epoxidharze zählen beispielsweise Polycarbonsäureanhydride, Komplexe von Aminen, insbesondere von primären oder tertiären aliphatischen aminen, wie beispielsweise Ethylamin, Trimethylamin oder n-Octyldimethylamin, mit $BF_3$ oder $BCl_3$, sowie latente Bordifluoridchelate. Aromatische Polyamine oder Imidazole sind in der Regel nicht bevorzugt, da mässige Resultate erhalten werden. Möglicherweise ist dies einer Reaktion zwischen dem freigesetzten sauren Katalysator und dem Amin zuzuschreiben.

Dicyandiamid kann erfolgreich eingesetzt werden, vorausgesetzt es liegt in einer relativ grobkörnigen Erscheinungsform vor.

Zu den geeigneten wärmeaktivierbaren Vernetzungsmitteln für Phenoplaste zählen beispielsweise Hexamethylentetramin und Paraformaldehyd.

Temperaturen und Zeitdauer der Erwärmung beim thermischen Aushärten nach der Photopolymerisation, sowie die Anteile der einzusetzenden wärmeaktivierbaren Härter, lassen sich anhand von Routinetests vom Fachmann ermitteln.

In den nachfolgenden Beispielen bedeuten Teile Gewichtsteile.

**Beispiel 1**

Herstellung von Dimethylmethansulfonylmethylsulfoxonium-hexafluorphosphat

Dimethylsulfoxoniummethylid wird nach der Methode von Corey und Chaykovsky (J. Am. Chem. Soc., 84, 867, 1962) hergestellt. Dazu setzt man 24 Teile Trimethylaulfoxoniumchlorid mit 6,24 Teilen Natriumhydrid (80 %ig in Öl) in 300 ml trockenem Tetrahydrofuran um. Diese 300 ml Ylidlösung (enthaltend 0,15 Mol Ylid) wird in Dimethylsulfoxoniummethansulfonylmethylid überführt, indem man 8,59 Teile Methansulfonylchlorid zufügt (Methode von Truce und Madding in Tetrahedron Lett., 1966, 3681).

Das Methansulfonylmethylid wird durch 5-minütiges Hindurchleiten von HCl-Gas durch die Lösung neutralisiert und die resultierende Lösung konzentriert.

Man fügt Diethylether zu und erhält einen feinverteilten weissen Feststoff. Diese Substanz wird abfiltriert, mit Diethylether gewaschen und im Vakuum getrocknet. Der Rückstand wird aus Methanol umkristallisiert und man erhält 10,24 Teile des Produktes Dimethylmethansulfonylmethylsulfoxoniumchlorid.

10,24 Teile dieser Verbindung werden in 50 Teilen Wasser gelöst und eine Lösung von 9,12 Teilen Kaliumhexafluorphosphat in 200 Teilen Wasser zugegeben. Die Lösung wird 30 Minuten bei Raumtemperatur gerührt und über Nacht im Kühlschrank stehen gelassen. Man erhält einen Niederschlag, der durch Absaugen des Wassers abgetrennt, mit einem geringen Anteil Methanol gewaschen, anschliessend mit Ether gewaschen und im Vakuum getrocknet wird. Man erhält 8,67 Teile eines Produktes mit dem F.P. 161-2°C.

$^1$H-NMR Spektrum ($d_6$-Aceton): 3,46 (s, 3H), 4,25 (s, 6H), 6,13 (s, 2H) (δ-Werte in ppm).

IR-Spektrum (KBr Platte): 3020, 2922, 1322, 1303, 1246, 1164, 840 (jeweils $cm^{-1}$).

**Beispiel 2**

Herstellung von Dimethylethansulfonylmethylsulfoxonium-hexafluorphosphat

Dimethylethansulfonylmethylsulfoxonium-hexafluorphosphat wird nach der in Beispiel 1 beschriebenen Methode hergestellt, indem man Ethansulfonylchlorid (9,64 Teile) anstelle von Methansulfonylchlorid einsetzt. Man erhält auf diese Weise 5,81 Teile des gewünschten Produktes vom F.P. 107-8°C.

$^1$H-NMR Spektrum ($d_6$-Aceton): 1,46 (t, 3H, J=7,2 Hz) 4,60 (q, 2H, J=7,0 Hz), 4,29 (s, 6H) 6,13 (s, 2H) (δ-Werte in ppm)

IR-Spektrum (KBr Platte) 3018, 2980, 2921, 1328, 1248, 1150, 843 (jeweils $cm^{-1}$).

**Beispiel 3**

Herstellung von Dimethylacetylmethylsulfoxonium-hexafluorphosphat

Dimethylsulfoxoniummethylid wird nach der Methode von Corey und Chaykovsky, wie in Beispiel 1 beschrieben, hergestellt. Dazu werden 24 Teile Trimethylsulfoxoniumchlorid mit 6,24 Teilen Natriumhydrid (80 %-ig in Öl) in 300 ml trockenem Tetrahydrofuran behandelt.

300 ml dieser Ylidlösung (0,16 Mol Ylid) werden in das Dimethylsulfoxoniumacetylmethylid überführt, indem

man 8,16 Teile Acetanhydrid zu besagter Ylidlösung zutropft. Das Zutropfen erfolgt unter Kühlung und über eine Zeitdauer von 10 Minuten. Die Reaktionslösung wird anschliessend 90 Minuten bei Raumtemperatur gerührt.

Das entstandene Dimethylsulfoxoniumacetylmethylid wird durch tropfenweise Zugabe von 12 Teilen Eisessig neutralisiert (Kühlung, Zeitdauer 5 Minuten), und die Mischung anschliassend bei Raumtemperatur 30 Minuten lang gerührt. Anschliessend filtriert man ab. Dem Filtrat wird Diethylether zugegeben und es wird nochmals filtriert. Das Filtrat wird bis zur Trockne eingeengt (Vakuumdestillation an einem Rotationsverdampfer) und der Rückstand in 30 Teilen Wasser aufgenommen.

Zu dieser Lösung fügt man 36,8 Teile Kaliumhexafluorphosphat in 500 Teilen Wasser und rührt die resultierende Lösung bei Raumtemperatur 30 Minuten lang. Die Reaktionslösung wird dann über Nacht im Kühlschrank stehen gelassen und der ausgefallene Niederschlag durch Absaugen abgetrennt. Das Produkt wird zunächst mit einer kleinen Menge Methanol, und anschliessend mit Diethylether gewaschen. Der Rückstand wird im Vakuum getrocknet. Man erhält 8,04 Teile des gewünschten Produktes vom F.P. 120-1°C.

$^1$H-NMR Spektrum ($d_6$-Aceton): 2,43 (s, 3H), 4,00 (s, 6H), 5,49 (s, 2H) (δ-Werte in ppm).

IR-Spektrum (KBr Platte): 3020, 2938, 2922, 1722, 1405, 1351, 1295, 1228, 1153, 1031, 825 (jeweils $cm^{-1}$).

**Beispiel 4**

Herstellung von n-Butylcarbamoylmethyldimethylsulfoxonium-hexafluorphosphat

Dimethylsulfoxoniummethylid wird nach der Methode von Corey und Chaykovsky, wie in Beispiel 1 beschrieben, hergestellt. Dazu werden 24 Teile Trimethylsulfoxoniumchlorid mit 6,24 Teilen Natriumhydrid (80 %-ig in Öl) in 300 ml trockenem Tetrahydrofuran behandelt. 300 ml dieser Ylidlösung (0,16 Mol Ylid) werden in das Diemthylsulfoxonium-n-butylcarbamoylmethylid überführt, indem man 15,84 Teile n-Butylisocyanat zu besagter Ylidlösung zutropft. Das Zutropfen erfolgt unter Kühlung und über eine Zeitdauer von 15 Minuten. Die Reaktionslösung wird anschliessend 90 Minuten bei Raumtemperatur gerührt.

Das entstandene Produkt wird abgesaugt und in 500 Teilen Wasser enthaltend 160 ml N-Salzsäure gelöst.

Zu dieser Lösung fügt man 29,4 Teile Kaliumhexafluorphosphat in 500 Teilen Wasser und rührt die resultierende Lösung bei Raumtemperatur 30 Minuten lang. Die Reaktionslösung wird dann über Nacht im Kühlschrank stehen gelassen und der ausgefallene Niederschlag durch Absaugen abgetrennt. Das Produkt wird zunächst mit einer kleinen Menge Methanol gewaschen, und anschliessend mit Diethylether. Der Rückstand wird im Vakuum getrocknet. Man erhält 11,2 Teile des gewünschten Produktes vom F.P. 87-8°C.

$^1$H-NMR Spektrum ($d_6$-Aceton): 0,70-1,70 (m, 7H), 3,30 (q, 2H), 4,05 (s, 6H), 5,12 (s, 2H), 7,90 (s, 1H) (δ-Werte in ppm).

IR Spektrum (KBr Platte): 3410, 3015, 2920, 1670, 1540, 1329, 1192, 1030, 830 (jeweils $cm^{-1}$).

**Beispiel 5**

Herstellung von Dimethylethoxycarbonylmethylsulfoxonium-hexafluorphosphat

Dimethylsulfoxoniummethylid wird nach der Methode von Corey und Chaykovsky, wie in Beispiel 1 beschrieben, hergestellt. Dazu werden 24 Teile Trimethylsulfoxoniumchlorid mit 6,24 Teilen Natriumhydrid (80 %-ig in Öl) in 300 ml trockenem Tetrahydrofuran behandelt.

300 ml dieser Ylidlösung (0,16 Mol Ylid) werden in das Dimethylsulfoxoniumethoxycarbonylmethylid überführt, indem man 8,68 Teile Ethylchloroformat zu besagter Ylidlösung zutropft. Das Zutropfen erfolgt unter Kühlung und über eine Zeitdauer von 10 Minuten. Die Reaktionslösung wird anschliessend 90 Minuten bei Raumtemperatur gerührt.

Das entstandene Dimethylsulfoxoniumethoxycarbonylmethylid wird durch Durchleiten von HCl Gas durch die Lösung neutralisiert (Zeitdauer 5 Minuten), und die Mischung anschliessend bei Raumtemperatur 30 Minuten lang gerührt. Anschliessend filtriert man ab. Dem Filtrat wird Diethylether zugegeben, wobei ein klebriger Feststoff ausfällt. Die überstehende Flüssigkeit wird abdekantiert und der Rückstand in Wasser aufgelöst.

Zu dieser Lösung fügt man 18,94 Teile Kaliumhexafluorphosphat in 400 Teilen Wasser und rührt die resultierende Lösung bei Raumtemperatur 30 Minuten lang. Die Reaktionslösung wird dann 16 Stunden lang im Kühlschrank stehen gelassen und der ausgefallene Niederschlag durch Absaugen abgetrennt. Das Produkt wird zunächst mit einer kleinen Menge Methanol, und anschliessend mit Diethylether gewaschen. Der Rückstand wird im Vakuum getrocknet. Man erhält 4,92 Teile des gewünschten Produktes vom F.P. 75-76°C.

$^1$H-NMR Spektrum ($d_6$-Aceton): 1,33 (s, 3H) 4,11 (s, 6H), 4,31 (q,2H), 5,39 (s 2H) (δ-Werte in ppm).

IR-Spektrum (KBr Platte): 3022, 2983, 2930, 1728,1405, 1334, 1317, 1240, 1190, 830 (jeweils $cm^{-1}$).

**Beispiel 6**

Herstellung von Dimethyltricyclo[3.3.1.1$^{3,7}$]decan-1-carbonylmethylsulfoxonium-hexafluorphosphat

Dimethylsulfoxoniummethylid wird nach der Methode von Corey und Chaykovsky, wie in Beispiel 1 beschrieben, hergestellt. Dazu werden 24 Teile Trimethylsulfoxoniumchlorid mit 6,24 Teilen Natriumhydrid (80 %-ig in Öl) in 300 ml trockenem Tetrahydrofuran behandelt.

300 ml dieser Ylidlösung (0,16 Mol Ylid) werden in das Dimethyl-sulfoxoniumtricyclo[3.3.1.1$^{3,7}$]decan-1-carbonylmethylid überfuhrt, indem man 15,88 Teile Tricyclo[3.3.1.1$^{3,7}$]decan-1-carbonsäure-chlorid zu besagter Ylidlösung zutropft. Das Zutropfen erfolgt portionsweise unter Kühlung und über eine Zeitdauer von 10 Minuten. Die Reaktionslösung wird anschliessend 90 Minuten bei Raumtemperatur gerührt.

Das entstandene Dimethylsulfoxoniumtricyclo[3.3.1.1$^{3,7}$]decan-1-carbonylmethylid wird durch Hindurchleiten von HCl Gas durch die Lösung neutralisiert (Zeitdauer 10 Minuten), und die Mischung anschliessend bei Raumtemperatur 30 Minuten lang gerührt. Anschliessend filtriert man ab. Dem Filtrat wird Diethylether zugegeben und der gebildete Niederschlag wird abgesaugt. Der Rückstand wird aus Methanol umkristallisiert und in Wasser aufgenommen.

Zu dieser Lösung fügt man eine Lösung von 14,72 Teilen Kaliumhexafluorphosphat und rührt die resultierende Mischung bei Raumtemperatur 30 Minuten lang. Der ausgefallene Niederschlag wird durch Absaugen abgetrennt. Das Produkt wird zunächst mit einer kleinen Menge Methanol, und anschliessend mit Diethylether gewaschen. Der Rückstand wird im Vakuum getrocknet. Man erhält 10,9 Teile des gewünschten Produktes vom F.P. 186-187° C.

$^1$H-NMR Spektrum ($d_6$-DMSO): 1,50-2,20 (m, 15H), 3,85 (s, 6H), 5,68 (s, 2H) (δ-Werte in ppm).

IR-Spektrum (KBr Platte): 3035, 2960, 2927, 2850, 1688, 1405, 1328, 1290, 1222, 1150, 1035, 1001, 981, 835 (jeweils $cm^{-1}$).

**Beispiel 7**

Eine Zusammensetzung bestehend aus 100 Teilen 2,2-Bis-(4-glycidyloxyphenyl)-propan, 4,7 Teilen Dimethylmethansulfonylmethylsulfoxonium-hexafluorphosphat und 5 Teilen Aceton wird als 6 µm dicker Film auf eine Weissblechplatte aufgetragen. Der Film wird der Strahlung einer 80 W Mitteldruck-Quecksilberdampflampe ausgesetzt, indem er in einer Entfernung von 8 cm von der Lampe in einem Primarc Apparat mit einer Geschwindigkeit von 2,1 m/min bewegt wird. Eine klebfreie Beschichtung wird nach vier Durchläufen erhalten.

**Beispiel 8**

Man wiederholt das Verfahren von Beispiel 7. Dabei enthält die Harzzusammensetzung noch zusätzlich 2,5 Teile Phenol. Nach drei Durchläufen unter der Lampe erhält man eine klebfreie Beschichtung der Platte.

**Beispiel 9**

Eine Zusammensetzung bestehend aus 100 Teilen 2,2-Bis-(4-glycidyloxyphenyl)-propan, 5 Teilen Dimethylethansulfonylmethylsulfoxonium-hexaflurophosphat und 5 Teilen Aceton wird als 6 µm dicker Film auf eine Weissblechplatte aufgetragen. Der Film wird der Strahlung einer 80 W Mitteldruck-Quecksilberlampe in einer Entfernung von 8 cm ausgesetzt. Nach 5 Sekunden ist eine klebfreie Beschichtung der Oberfläche entstanden.

**Beispiel 10**

Das Verfahren von Beispiel 9 wird wiederholt, indem man eine Zusammensetzung verwendet, die zusätzlich 2,5 Teile Phenol enthält. Nach 5 Sekunden ist eine klebfreie Beschichtung der Oberfläche entstanden.

**Beispiel 11**

Eine Zusammensetzung bestehend aus 100 Teilen (2,3-Dihydro-4H-pyran-2-yl)-methyl-2,3-dihydro-4H-pyran-2-carboxylat, 5 Teilen Dimethylethansulfonylmethylsulfoxonium-hexafluorphosphat und 5 Teilen Aceton wird als 6 µm dicker Film auf eine Weissblechplatte aufgetragen. Der Film wird der Strahlung einer 80 W Mitteldruck-Quecksilberlampe in einer Entfernung von 8 cm ausgesetzt. Nach 30 Sekunden ist eine klebfeie Beschichtung der Oberfläche entstanden.

**Beispiel 12**

Das Verfahren von Beispiel 11 wird wiederholt, indem man eine Zusammensetzung verwendet, die zusätzlich 2,5 Teile Phenol enthält. Nach 23 Sekunden ist eine klebfreie Beschichtung der Oberfläche entstanden.

**Beispiel 13**

Eine Zusammensetzung bestehend aus 100 Teilen 3,4-Epoxycyclohexylmethyl-3',4'-epoxycyclohexancarboxylat, 5 Teilen Dimethylethansulfonylmethylsulfoxonium-hexafluorphosphat und 5 Teilen Aceton wird als 6 µm dicker Film auf eine Weissblechplatte aufgetragen. Der Film wird der Strahlung einer 80 W Mitteldruck-Quecksilberlampe in einer Entfernung von 8 cm ausgesetzt. Nach 60 Sekunden ist der Film noch klebrig.
Verwendet man eine Zusammensetzung wie oben, die zusätzlich 2,5 Teile Phenol enthält, so ist nach 14 Sekunden eine klebfreie Beschichtung der Oberfläche entstanden.

**Beispiel 14**

Eine Zusammensetzung bestehend aus 100 Teilen 2,2-Bis-(4-glycidyloxyphenyl)-propan, 4,2 Teilen Dimethylacetylmethylsulfoxonium-hexafluorphosphat und 5 Teilen Aceton wird als 6 µm dicker Film auf eine Weissblechplatte aufgetragen. Der Film wird der Strahlung einer 80 W Mitteldruck-Quecksilberlampe in einer Entfernung von 8 cm ausgesetzt. Nach 10 Sekunden ist eine klebfreie Beschichtung der Oberfläche entstanden.

**Beispiel 15**

Das Verfahren von Beispiel 14 wird wiederholt, indem man eine Zusammensetzung verwendet, die zusätzlich 2,5 Teile Phenol enthält. Nach 8 Sekunden ist eine klebfreie Beschichtung der Oberfläche entstanden.

**Beispiel 16**

Eine Zusammensetzung bestehend aus 100 Teilen (2,3-Dihydro-4H-pyran-2-yl)-methyl-2,3-dihydro-4H-pyran-2-carboxylat, 4,2 Teilen Dimethylacetylmethylsulfoxonium-hexafluorphosphat und 5 Teilen Aceton wird als 6 µm dicker Film auf eine Weissblechplatte aufgetragen. Der Film wird der Strahlung einer 80 W Mitteldruck-Quecksilberlampe in einer Entfernung von 8 cm ausgesetzt. Nach 35 Sekunden ist eine klebfreie Beschichtung der oberfläche entstanden.

**Beispiel 17**

Das Verfahren von Beispiel 16 wird wiederholt, indem man eine Zusammensetzung verwendet, die zusätzlich 2,5 Teile Phenol enthält. Nach 20 Sekunden ist eine klebfreie Beschichtung der Oberfläche entstanden.

**Beispiel 18**

Eine Zusammensetzung bestehend aus 100 Teilen 3,4-Epoxycyclohexylmethyl-3',4'-epoxycyclohexancarboxylat, 4,2 Teilen Dimethylacetylmethylsulfoxonium-hexafluorphosphat und 5 Teilen Aceton wird als 6 µm dicker Film auf eine Weissblechplatte aufgetragen. Der Film wird der Strahlung einer 80 W Mitteldruck-Quecksilberlampe in einer Entfernung von 8 cm ausgesetzt. Nach 55 Sekunden ist eine klebfreie Beschichtung der Oberfläche entstanden.

**Beispiel 19**

Das Verfahren von Beispiel 18 wird wiederholt, indem man eine Zusammensetzung verwendet, die zusätzlich 2,5 Teile Phenol enthält. Nach 25 Sekunden ist eine klebfreie Beschichtung der Oberfläche entstanden.

**Beispiel 20**

Eine Zusammensetzung bestehend aus 100 Teilen eines Phenol-Formaldehyd Resols mit einem Phenol/Formaldehyd Verhältnis von 1:1,5, aus 5 Teilen Dimethylethansulfonylmethylsulfoxonium-hexafluorphosphat und 5 Teilen Aceton wird als 6 µm dicker Film auf eine Weissblechplatte aufgebracht. Der Film wird der Strahlung einer 80 W Mitteldruck-Quecksilberlampe in einer Entfernung von 8 cm ausgesetzt. Die Bestrahlungsdauer beträgt insgesamt 30 Sekunden (jeweils drei Perioden von je 10 Sekunden gefolgt von einer Abkühlphase zwischen den einzelnen Belichtungen).

Die König-Härte dieser Beschichtung beträgt 45,1 Sekunden.

**Beispiel 21**

Eine Zusammensetzung bestehend aus 100 Teilen eines Melamin-Formaldehyd Harzes mit einem Melamin/Formaldehyd Verhältnis von 1:4,5, aus 5 Teilen Dimethylethansulfonylmethylsulfoxonium-hexafluorphosphat und 5 Teilen Aceton wird als 6 µm dicker Film auf eine Weissblechplatte aufgebracht. Der Film wird der Strahlung einer 80 W Mitteldruck-Quecksilberlampe in einer Entfernung von 8 cm ausgesetzt. Die Bestrahlungsdauer beträgt insgesamt 30 Sekunden (jeweils drei Perioden von je 10 Sekunden gefolgt von einer Abkühlphase zwischen den einzelnen Belichtungen).

Die König-Härte dieser Beschichtung beträgt 94,6 Sekunden.

**Beispiel 22**

Eine Zusammensetzung bestehend aus 100 Teilen 2,2-Bis[4-(2-hydroxy-3-methacryloyloxy-propoxy)-phenyl]-propan, 4,7 Teilen Dimethylmethansulfonylmethylsulfoxonium-hexafluorphosphat und 5 Teilen Aceton wird als 6 µm dicker Film auf eine Weissblechplatte aufgetragen. Der Film wird der Strahlung einer 80 W Mitteldruck-Quecksilberdampflampe ausgesetzt, indem er in einer Entfernung von 8 cm von der Lampe in einem Primarc Apparat mit einer Geschwindigkeit von 2,1 m/min bewegt wird. Eine klebfreie Beschichtung wird nach drei Durchläufen erhalten.

**Beispiel 23**

Beispiel 22 wird wiederholt, indem man n-Butylcarbamoylmethyldimethylsulfoxonium-hexafluorphosphat (4,8 Teile) anstelle von Dimethylmethansulfonylmethylsulfoxonium-hexafluorphosphat verwendet. Eine klebfreie Beschichtung wird nach 4 Durchläufen erhalten.

**Beispiel 24**

Eine Zusammensetzung bestehend aus 100 Teilen 2,2-Bis-(4-glycidyloxyphenyl)-propan, 4,7 Teilen Dimethylethoxycarbonylmethylsulfoxonium-hexafluorphosphat und 5 Teilen Aceton wird als 6 µm dicker Film auf einer Zinnplatte aufgetragen. Der Film wird der Strahlung einer 80 W Mitteldruck-Quecksilberlampe in

einer Entfernung von 8 cm ausgesetzt. Nach 10 Sekunden ist eine klebfreie Beschichtung der Oberfläche entstanden.

**Beispiel 25**

Eine Zusammensetzung bestehend aus 100 Teilen 2,2-Bis-(4-glycidyloxyphenyl)-propan, 6,0 Teilen Dimethyltricyclo-[3.3.1.1$^{3,7}$)decan-1-carbonylmethylsulfoxonium-hexafluorphosphat und 5 Teilen Aceton wird als 6 μm dicker Film auf einer Zinnplatte aufgetragen. Der Film wird der Strahlung einer 80 W Mitteldruck-Quecksilberlampe in einer Entfernung von 8 cm ausgesetzt. Nach 5 Sekunden ist eine klebfreie Beschichtung der Oberfläche entstanden.

**Beispiel 26**

Eine. Zusammensetzung bestehend aus 100 Teilen 2,2-Bis-(4-glycidyloxyphenyl)-propan, 5,0 Teilen Dimethyltricyclo[3.3.1.1$^{3,7}$]decan-1-carbonylmethylsulfoxonium-hexafluorphosphat und 5 Teilen Aceton wird als 6 μm dicker Film auf einer Zinnplatte aufgetragen. Der Film wird der Strahlung einer 15 W Niederdruck-Quecksilberlampe in einer Entfernung von 2 cm ausgesetzt (Emissionsmaximum: 254 nm). Nach 1 Minute ist eine klebfreie Beschichtung der Oberfläche entstanden.

**Beispiel 27**

Eine Zusammensetzung bestehend aus 100 Teilen 2,2-Bis-(4-glycidyloxyphenyl)-propan, 5 Teilen Dimethyltricyclo-[3.3.1.1$^{3,7}$]decan-1-carbonylmethylsulfoxonium-hexafluorphosphat und 5 Teilen Aceton wird als 40 μm dicker Film auf einer Zinnplatte aufgetragen. Der film wird der Strahlung einer 15 W Niederdruck-Quecksilberlampe in einer Entfernung von 2 cm ausgesetzt (Emissionsmaximum:254 nm). Nach 90 Sekunden ist eine klebfreie Beschichtung der Oberfläche entstanden.

**Beispiel 28**

4,7 Teile Dimethylmethansulfonylmethylsulfoxonium-hexafluorphosphat werden mit 5 Teilen Aceton gemischt und anschliessend zu 100 Teilen Polyglycidylether von 2,2-Bis-(4-hydroxyphenyl)-propan mit einem Epoxidgehalt von 5,2 Äquivalenten/kg und 5 Teilen 70 %-igem Benzoylperoxid gegeben. Die Zusammensetzung wird auf einer Kofler Bank auf 120°C aufgeheizt, und eine Gelierungszeit von 14 Minuten wird ermittelt.

**Beispiel 29**

Das Verfahren von Beispiel 28 wird wiederholt, indem man 5 Teile Kupfer-acetylacetonat anstelle des 70 %-igen Benzoylperoxids einsetzt. Man misst eine Gelierungszeit von 8 Minuten.

**Beispiel 30**

5 Teile Dimethylethansulfonylmethylsulfoxonium-hexafluorphosphat und 5 Teile Aceton werden miteinander gemischt. Anschliessend gibt man dieses Gemisch zu 100 Teilen eines Polyglycidylethers von 2,2-Bis-(4-hydroxdyphenyl)-propan mit einem Epoxidgehalt von 5,2 Äquivalenten/kg. Die Zusammensetzung wird auf einer Kofler Bank auf 120°C aufgeheizt. Nach 65 Minuten geliert die Mischung.

Wiederholt man den oberen Test mit einer Mischung enthaltend zusätzlich 5 Teile 70 %-iges Benzoylperoxid, so misst man eine Gelierungszeit von 11 Minuten.

**Beispiel 31**

4,2 Teile Dimethylacetylmethylsulfoxonium-hexafluorphosphat und 5 Teile Aceton werden gemischt. Diese Mischung gibt man zu 100 Teilen eines Polyglycidylethers von 2,2-Bis-(4-hydroxyphenyl)-propan mit einem Epoxidgehalt von 5,2 Äquivalenten/kg und zu 5 Teilen 70 %-igem Benzoylperoxid.

Die Zusammensetzung wird auf einer Kofler Bank auf 120°C aufgeheizt und man misst eine Gelierungszeit von 9 Minuten.

**Beispiel 32**

4,7 Teile Dimethylethoxycarbonylmethylsulfoxonium-hexafluorphosphat werden mit 5 Teilen Aceton gemischt und anschliessend zu 100 Teilen Polyglycidylether von 2,2-Bis-(4-hydroxy-phenyl)-propan mit einem Epoxidgehalt von 5,2 Äquivalenten/kg und 5 Teilen 70 %-igem Benzoylperoxid gegeben. Die Zusammensetzung wird auf einer Kofler Bank auf 120°C aufgeheizt, und eine Gelierungszeit von 11,5 Minuten wird ermittelt.

**Patentansprüche**

1. Aliphatische Sulfoxoniumsalze der Formel I

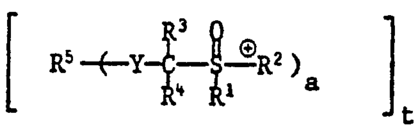 a [ $Z^{t-}$ ] (I),

worin $R^1$ $C_1$-$C_{18}$ Alkyl, $C_2$-$C_6$ Alkenyl, $C_3$-$C_8$ Cycloalkyl oder $C_4$-$C_{10}$ Cycloalkylalkyl bedeutet,

$R^2$ eine der Bedeutungen von $R^1$ besitzt oder zusätzlich $C_2$-$C_6$ Dialkylamino ist, worin $R^3$ und $R^4$ unabhängig voneinander Wasserstoff, $C_1$-$C_{25}$ Alkyl oder eine der Gruppen -$COR^6$, -CO-NH-$(CO)_b$ -$R^6$ oder -$SO_2$-$R^6$ darstellen, worin

$R^6$ gesättigtes oder ethylenisch ungesättigtes $C_1$-$C_{25}$ Alkyl bedeutet, worin

$R^5$ ein mono- bis tetravalenter gesättigter oder ungesättigter aliphatischer $C_1$-$C_{25}$ Rest ist, ein cycloaliphatischer Rest mit bis zu 25 Kohlenstoffatomen ist oder einen $C_1$-$C_{25}$ Alkoxyrest darstellt, worin

Y eine Carbonylgruppe, eine Sulfonylgruppe oder einen Rest der Formel -$(CO)_b$ -NH-CO- darstellt,

a eine ganze Zahl von 1 bis 4 ist,

b 0 oder 1 bedeutet,

t 1, 2 oder 3 ist,

$Z^{t-}$ ein t-wertiges Anion ist, und ein Hydroxypentafluorantimonatanion ist, oder ein Anion der Formel $R^7$-$(SO_3^-)_t$ oder $MX^-_c$,

$R^7$ Wasserstoff oder ein ein-, zwei- oder dreiwertiger aliphatischer, aromatischer oder aliphatisch-substituierter aromatischer $C_1$-$C_{25}$ Rest ist,

M ein Metall oder Metalloidatom bedeutet,

X ein Halogenatom ist, und worin schliesslich

c 4, 5 oder 6 ist, und um eins grösser ist, als die Valenz von M.

2. Sulfoxoniumsalze der Formel I gemäss Anspruch 1, worin $R^1$ und $R^2$ $C_1$-$C_4$ Alkyl sind.

3. Sulfoxoniumsalze der Formel I gemäss Anspruch 1, worin $R^3$ und $R^4$ Wasserstoff oder $C_1$-$C_4$ Alkyl bedeuten.

4. Sulfoxoniumsalze der Formel I gemäss Anspruch 1, worin $R^5$ $C_1$-$C_4$ Alkyl, $C_1$-$C_4$ Alkoxy oder ein Cycloalkylrest mit bis zu 12 Kohlenstoffatomen ist.

5. Sulfoxoniumsalze der Formel I gemäss Anspruch 1, worin $R^7$ Wasserstoff, Methyl, Trifluormethyl, Phenyl, Tolyl, 1,4-Phenylen oder der Rest eines sulfonierten Phenol-Formaldehyd Novolaks nach dem Entfernen von 1, 2 oder 3 Sulfonsäuregruppen ist.

6. Sulfoxoniumsalze der Formel I gemäss Anspruch 1, worin M ein Atom ausgewählt aus der Gruppe Bor, Wismut, Antimon, Arsen oder Phosphor darstellt.

7. Sulfoxoniumsalze der Formel I gemäss Anspruch 6, worin das Anion $Z^{t-}$ ausgewahlt wird aus der Gruppe bestehend aus $BiCl^-_6$, $BF^-_4$, $PF^-_6$, $SbF^-_6$ oder $AsF^-_6$.

8. Verfahren zur Herstellung von Sulfoxoniumsalzen der Formel I gemäss Anspruch 1, dadurch gekennzeichnet, dass man ein Oxosulfoniumylid der Formel II

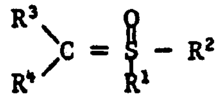

(II)

mit

a) einem Säurehalogenid der Formel III

$$R^5 \leftarrow CO\text{-}Hal)_a \qquad (III)$$

$$R^5 \leftarrow COOH)_a \qquad (IV);$$

oder mit

b) einem Sulfonylhalogenid der Formel V,

$$R^5 \leftarrow SO_2\text{-}Hal)_a \qquad (V);$$

oder mit

c) einem Isocyanat der Formel VI

$$R^5 \leftarrow [(CO)_b\text{-}NCO]_a \qquad (VI),$$

umsetzt, und anschliessend das resultierende Produkt einer der obigen Reaktionen entweder mit einer Säure der Formel $H_tZ$ neutralisiert oder besagtes resultierendes Produkt mit einer anderen Säure neutralisiert und das resultierende Salz anschliessend einer weiteren Umsetzung mit einem Metall- oder Ammoniumsalz des Anions $Z^{t-}$ unterwirft, wobei die Reste $R^1$, $R^2$, $R^3$, $R^4$, $R^5$ und $Z^{t-}$ sowie die Indizes, a, b und t die in Anspruch 1 definierte Bedeutung besitzen.

9. Zusammensetzung enthaltend

a) eine Verbindung, die sich unter dem Einfluss eines Salzes der Formel I gemäss Anspruch 1 polymerisieren lässt, und

b) eine wirksame Menge eines aliphatischen Sulfoxoniumsalzes, wie in Anspruch 1 definiert.

10. Zusammensetzung gemäss Anspruch 9, enthaltend von 0,1 bis 7,5 Gewichtsteile der Komponente b) auf 100 Gewichtsteile der Komponente a).

11. Zusammensetzung gemäss Anspruch 9, worin Komponente a) ein 1,2-Epoxid, ein Vinylmonomeres oder dessen Präpolymeres, ein Aminoplast oder ein Phenoplast ist.

12. Zusammensetzung gemäss Anspruch 11, worin Komponente a) eine Epoxidverbindung ist, die mindestens einen aromatischen Ring enthält.

13. Zusammensetzung gemäss Anspruch 9, enthaltend zusätzlich einen aromatischen Sensibilisator.

14. Zusammensetzung gemäss Anspruch 13, worin der aromatische Sensibilisator eine aromatische Verbindung ist, die eine Triplett-Energie von mehr als 75 kcal/Mol besitzt.

15. Zusammensetzung gemäss Anspruch 9, die zusätzlich einen Aktivator für das Sulfoxoniumsalz der Formel I enthält.

16. Zusammensetzung gemäss Anspruch 15, worin der Aktivator ein Salz oder ein Komplex eines Übergangsmetalls, ein Zinnsalz, ein organisches Peroxid oder eine aktivierte α-Hydroxyverbindung bedeutet.

17. Zusammensetzung gemäss Anspruch 9, worin Komponente b) eine Verbindung ist aus der Gruppe bestehend aus Dimethylmethansulfonylmethylsulfoxonium-hexafluorphosphat, Dimethylethansulfonylmethylsulfoxonium-hexafluorphosphat, Dimethylacetylmethylsulfoxonium-hexafluorphosphat, n-Butylcarbamoylmethyldimethylsulfoxonium-hexafluorphosphat, Dimethylethoxycarbonylmethylsulfoxonium-hexafluorphosphat oder Dimethyltricyclo[3.3.1.1$^{3,7}$]decan-1-carbonylmethylsulfoxonium-hexafluorphosphat.

**Claims**

1. Aliphatic sulphoxonium salts of the general formula I

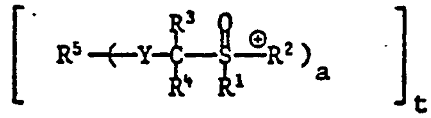 a [ $Z^{t-}$ ] (I),

in which $R^1$ is $C_1$-$C_{18}$-alkyl, $C_2$-$C_6$-alkenyl, $C_3$-$C_8$-cycloalkyl, or $C_4$-$C_{10}$-cycloalkylalkyl; $R^2$ has one of the meanings of $R^1$ or is also $C_2$-$C_6$-dialkylamino; $R^3$ and $R^4$ are independently of each other hydrogen, $C_1$-$C_{25}$-alkyl or one of the groups -$COR^6$, -CO-NH-$(CO)_b$-$R^6$ or -$SO_2$-$R^6$ where

$R^6$ is saturated or ethylenically unsaturated $C_1$-$C_{25}$-alkyl;

$R^5$ is a mono- to tetra-valent saturated or un-saturated aliphatic $C_1$-$C_{25}$-radical, a cycloaliphatic radical having up to 25 carbon atoms, or $C_1$-$C_{25}$-alkoxy radical; Y is a carbonyl group, a sulphonyl group or a radical of the formula -$(CO)_b$-NH-CO-;

a is an integer from 1 to 4;

b is 0 or 1; t is 1, 2 or 3;

$Z^{t-}$ is a t-valent anion which is a hydroxypentafluoroantimonate anion or an anion of the formula $R^7$ $(SO_3^-)_t$ or $MX^-_c$ where:

$R^7$ is hydrogen, or a mono-, di- or tri-valent aliphatic, aromatic or aliphatic-substituted aromatic $C_1$-$C_{25}$-radical;

M is a metal or metalloid atom;

X is a halogen atom, and finally c is 4, 5 or 6 and is one more than the valency of M.

2. Sulphoxonium salts of the formula I as claimed in Claim 1 in which $R^1$ and $R^2$ are $C_1$-$C_4$-alkyl.

3. Sulphoxonium salts of the formula I as claimed in Claim 1 in which $R^3$ and $R^4$ are hydrogen or $C_1$-$C_4$-alkyl.

4. Sulphoxonium salts of the formula I as claimed in Claim 1 in which $R^5$ is $C_1$-$C_4$-alkyl, $C_1$-$C_4$-alkoxy or a cycloalkyl radical having up to 12 carbon atoms.

5. Sulphoxonium salts of the formula I as claimed in Claim 1 in which $R^7$ is hydrogen, methyl, trifluoromethyl, phenyl, tolyl, 1,4-phenylene or the residue of a sulphonated phenol-formaldehyde novolak after removal of 1, 2 or 3 sulphonic acid groups.

6. Sulphoxonium salts of the formula I as claimed in Claim 1 in which M is an atom selected from the group boron, bismuth, antimony, arsenic or phosphorus.

7. Sulphoxonium salts of the formula I as claimed in Claim 6 in which the anion $Z^{t-}$ is selected from the group consisting of $BiCl^-_6$, $BF^-_4$, $PF^-_6$, $SbF^-_6$, or $AsF^-_6$.

8. A process for the preparation of sulphoxonium salts of the formula I as claimed in Claim 1 which comprises reacting an oxosulphonium ylide of the formula II

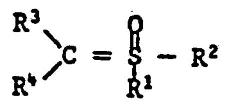 (II)

with

a) an acid halide of the formula III

$$R^5-(-CO-Hal)_a \quad (III)$$

or an anhydride of an acid of the formula IV

$$R^5-(-COOH)_a \quad (IV);$$

b) a sulphonyl halide of the formula V

$$R^5-(-SO_2-Hal)_a \quad (V);$$

or

c) an isocyanate of the formula VI

$$R^5 \text{-}[\text{-}(CO)_b\text{-}NCO]_a \qquad (VI),$$

and then either neutralising the resulting product of any of the above reactions with an acid of formula $H_tZ$, or neutralizing said resulting product with another acid and subsequently subjecting the resulting salt to a further reaction with a metal or ammonium salt of the anion $Z^{t-}$, wherein the radicals $R^1$, $R^2$, $R^3$, $R^4$, $R^5$ and $Z^{t-}$ as well as the indices a, b and t are as defined in Claim 1.

9. A composition containing

(a) a compound capable of being polymerised under the influence of a salt of the formula I as claimed in Claim 1, and

(b) an effective amount of an aliphatic sulphoxonium salt as defined in Claim 1.

10. A composition as claimed in Claim 9 which contains from 0.1 to 7.5 parts by weight of component (b) per 100 parts by weight of component (a).

11. A composition as claimed in Claim 9 in which component (a) is a 1,2-epoxide, a vinyl monomer or prepolymer thereof, an aminoplast or a phenoplast.

12. A composition as claimed in Claim 11 in which component (a) is an epoxy compound containing at least one aromatic ring.

13. A composition as claimed in Claim 9 which also contains an aromatic sensitiser.

14. A composition as claimed in Claim 13 in which the aromatic sensitiser is an aromatic compound with a triplet energy of more than 75 kcal/mole.

15. A composition as claimed in Claim 9 which also contains an activator for the sulphoxonium salt of the formula I.

16. A composition as claimed in Claim 15 in which the activator is a salt or a complex of a transition metal, a tin salt, an organic peroxide or an activated α-hydroxy compound.

17. A composition as claimed in Claim 9 in which component (b) is a compound from the group consisting of dimethylmethanesulphonylmethylsulphoxonium hexafluorophosphate, dimethylethanesulphonylmethylsulphoxonium hexafluorophosphate, dimethylacetylmethylsulphoxonium hexafluorophosphate, n-butylcarbamoylmethyldimethylsulphoxonium hexafluorophosphate, dimethylethyoxycarbonylmethylsulphoxonium hexafluorophosphate, or dimethyltricyclo-[3.3.1.1$^{3,7}$)decane-1-carbonylmethylsulphoxonium hexafluorophosphate.

## Revendications

1. Sels de sulfoxoniums aliphatiques répondant à la formule I:

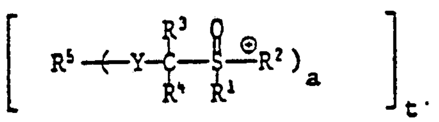
a [ $Z^{t-}$ ] (I)

dans laquelle :

$R^1$ représente un alkyle en $C_1$ -$C_{18}$, un alcényle en $C_2$-$C_6$, un cycloalkyle en $C_3$-$C_8$ ou un cycloalkyl-alkyle en $C_4$-$C_{10}$,

$R^2$ a l'une des significations de $R^1$ et peut en outre représenter un radical dialkylamino en $C_2$-$C_6$,

$R^3$ et $R^4$ représentent chacun, indépendamment l'un de l'autre l'hydrogène, un alkyle en $C_1$-$C_{25}$ ou l'un des radicaux -$COR^6$, -CO-NH-$(CO)_b$-$R^6$ et-$SO_2$-$R^6$,

$R^6$ représente un alkyle en $C_1$-$C_{25}$ saurté ou éthylénique,

$R^5$ représente un radical aliphatique en $C_1$ -$C_{25}$, saturé ou insaturé, et dont la valence peut aller de 1 à 4, un radical cycloaliphatique contenant au plus 25 atomes de carbone ou un radical alcoxy en $C_1$ -$C_{25}$,

Y représente un radical carbonyle, un radical sulfonyle ou un radical -$(CO)_b$,-CO-,

a représente un nombre entier de 1 à 4,

b représente le nombre 0 ou le nombre 1,

t représente l'un des nombres 1, 2 et 3,

$Z^{t-}$ représente un anion de valence t et est un anion hydroxy-pentefluoro-antimoniete ou un anion de formule $R^7$-$(SO_3^-)_t$ ou $MX^-_c$,

$R^7$ représente l'hydrogène ou un radical aliphatique, aromatique ou aromatique à substituant(s) aliphatique(s), en $C_1$-$C_{25}$, dont la valence est égale à 1, à 2 ou à 3,

M représente un atome de métal ou de métalloïde,

X représente un atome d'halogène et

c est égal à 4, à 5 ou à 6 et est supérieur d'une unité à la valence de M.

2. Sels de sulfoxoniums de formule I selon la revendication I dans lesquels $R^1$ et $R^2$ représentent chacun un alkyle en $C_1$-$C_4$.

3. Sels de sulfoxoniums de formule I selon la revendication I dans lesquels $R^3$ et $R^4$ représentent chacun l'hydrogène ou un alkyle en $C_1$-$C_4$.

4. Sels de sulfoxoniums de formule I selon la revendication 1 dans lesquels $R^5$ représente un alkyle en $C_1$-$C_4$, un alcoxy en $C_1$-$C_4$ ou un cycloalkyle contenant au plus 12 atomes de carbone.

5. Sels de sulfoxoniums de formule I selon la revendication 1 dans lesquels $R^7$ représente l'hydrogène, un radical méthyle, trifluorométhyle, phényle, tolyle ou phénylène-1,4, ou le radical qui subsiste lorsque, d'une novolaque phénol/formaldéhyde sulfonée, on a éliminé un, deux ou trois radicaux sulfo.

6. Sels de sulfoxoniums de formule I selon la revendication 1 dans lesquels M représente un atome pris dans l'ensemble constitué par le bore, le bismuth, l'antimoine, l'arsenic et le phosphore.

7. Sels de sulfoxoniums de formule I selon la revendication 6 dans lesquels l'anion $Z^{t-}$ est pris dans l'ensemble constitué par $BiCl_6^-$, $BF_4^-$, $PF_6^-$, $SbF_6^-$, et $AsF_6^-$.

8. Procédé de préparation de sels de sulfoxoniums de formule I selon la revendication 1, procédé caractérisé en ce qu'on fait réagir un ylide d'oxosulfonium de formule II:

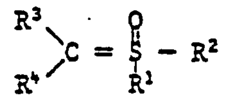 (II)

avec

a) un halogénure d'acide de formule III:

$$R^5 \text{-(} CO\text{-}Hal)_a \qquad (III)$$

ou un anhydride d'un acide de formule IV:

$$R^5 \text{-(} COOH)_a \qquad (IV);$$

ou avec

b) un halogénure de sulfonyle de formule V:

$$R^5 \text{-(} SO_2\text{-}Hal)_a \qquad (V);$$

ou avec

c) un isocyanate de formule VI:

$$R^5 \text{-[} (CO)_b\text{-}NCO]_a \qquad (VI),$$

formules dans lesquelles $R^1$, $R^2$, $R^3$, $R^4$, $R^5$ et $Z^{t-}$ ainsi que les indices a, b et t ont les significations données à la revendication 1, puis on neutralise le produit provenant de l'une des réactions précédentes soit avec un acide de formule $H_t Z$, soit avec un autre acide, après quoi on soumet le sel formé à une autre réaction avec un sel de métal ou d'ammonium de l'anion $Z^{t-}$.

9. Composition qui contient:

a) un composé capable de se polymériser sous l'action d'un sel de formule I selon la revendication 1 et

b) une quantité efficace d'un sel de sulfoxonium aliphatique tel que défini à la revendication 1.

10. Composition selon la revendication 9 qui contient de 0,1 à 7,5 parties en poids de la composante b) pour 100 parties en poids de la composante a).

11. Composition selon la revendication 9 dans laquelle la composante a) est un époxyde-1,2, un monomère vinylique ou l'un de ses prépolymères, un aminoplaste ou un phénoplaste.

12. Composition selon la revendication 11 dans laquelle la composante a) est un composé époxydique contenant au moins un cycle aromatique.

13. Composition selon la revendication 9 qui contient, en plus, un sensibilisateur aromatique.

14. Composition selon la revendication 13 dans laquelle le sensibilisateur aromatique est un composé aromatique qui a une énergie de triplet supérieure à 75 kcal/mol.

15. Composition selon la revendication 9 qui contient, en plus, un activeur pour le sel de sufoxonium de formule I.

16. Composition selon la revendicaion 15 dans laquelle l'activeur est un sel ou un complexe d'un métal de transition, un sel d'étain, un peroxyde organique ou un composé α-hydroxylique activé.

17. Composition selon la revendication 9 dans laquelle la composente b) est un compose pris dans l'ensemble constitué par l'hexafluorphosphate de diméthyl-(méthanesulfonylméthyl)-sulfoxonium, l'hexafluorophosphate de diméthyl-(éthanesulfonylméthyl)-sulfoxonium, l'hexafluorophosphate de diméthyl-(acétylméthyl)-sulfoxonium, l'hexafluorophosphate de (n-butylcarbamoylméthyl)-diméthylsulfoxonium, l'hexafluorophosphate de diméthyl-(éthoxycarbonylméthyl)-sulfoxonium et l'hexafluorophosphate de diméthyl-((tricyclo[3.3.1.1$^{3,7}$]decyl-1)-carbonylméthyl)-sulfoxonium.